Europäisches Patentamt

European Patent Office

Office européen des brevets

(19)

(11) Publication number: **0 211 381**
A1

(12) **EUROPEAN PATENT APPLICATION**

(21) Application number: **86110456.0**

(22) Date of filing: **29.07.86**

(51) Int. Cl.⁴: **C 07 C 7/163**
**C 07 C 11/08, C 07 C 11/09**

(30) Priority: **30.07.85 US 760371**

(43) Date of publication of application:
**25.02.87 Bulletin 87/9**

(84) Designated Contracting States:
**AT BE DE FR GB IT NL SE**

(71) Applicant: **PHILLIPS PETROLEUM COMPANY**
**5th and Keeler**
**Bartlesville Oklahoma 74004(US)**

(72) Inventor: **Rohr, Donald Frank, Jr.**
**15 Benny St., Apt 2**
**Newark Delaware 19711(US)**

(72) Inventor: **Haskell, Donald Mackintosh**
**1628 S.E. Osage**
**Bartlesville Oklahoma 74003(US)**

(72) Inventor: **Brinkmeyer, Francis Maurice**
**2124 S.E. Skyline Drive**
**Bartlesville Oklahoma 74006(US)**

(74) Representative: **Dost, Wolfgang, Dr.rer.nat.,**
**Dipl.-Chem. et al,**
**Patent- und Rechtsanwälte**
**Bardehle-Pagenberg-Dost-Altenburg & Partner Postfach**
**86 06 20**
**D-8000 München 86(DE)**

(54) **Hydrocarbon purification.**

(57) Diolefins present in minor amounts in hydrocarbon feed streams containing other unsaturated hydrocarbons, especially mono-olefins, are selectively converted to mono-olefins by hydrogenation over a steam active catalyst comprising a Group VIII metal, at least one of lead, tin and germanium, and a support, such as zinc aluminate.

EP 0 211 381 A1

Croydon Printing Company Ltd.

1

# HYDROCARBON PURIFICATION

## Background of the Invention

This invention relates to the selective hydrogenation of unsaturated materials in streams containing unsaturated contaminants. In accordance with another aspect, this invention relates to the selective hydrogenation of diolefins in hydrocarbon streams containing mono-olefins. In accordance with a further aspect, this invention relates to the selective hydrogenation of butadiene contained in commercial streams containing unsaturated hydrocarbons by contacting with a supported Group VIII metal catalyst promoted with at least one of lead, tin and germanium. In accordance with a further aspect, this invention relates to the selective hydrogenation of a diolefin, such as butadiene, contained in unsaturated hydrocarbon streams without substantial alteration of the mono-olefinic hydrocarbons present in the stream by contacting with a catalyst containing platinum, tin and a support, such as zinc aluminate.

The invention is also applied to the selective hydrogenation treatment of other impurities present in unsaturated hydrocarbon streams, such as acetylenes, sulfur compounds, eg. mercaptans, and other unidentified impurities which react with hydrogen. Thus, the invention specifically relates to a process for purifying $C_4$ hydrocarbon streams, e.g., 1-butene-containing streams.

In petroleum processing, a wide variety of materials differing appreciably as to origin are found in petroleum streams. For example, as a result of various processing steps certain petroleum streams have been found to contain unsaturated contaminants which need to be removed prior to further processing of the streams. The elimination of such materials from such streams generally involves a plurality of steps each affecting one or more of such materials in a manner to simplify their subsequent removal from the stock.

In the working-up of products obtained by the conversion of hydrocarbon oils, fractions are produced that contain primarily $C_4$ hydrocarbon, such as n-butane, n-butene-1, cis-butene-2, trans-butene-2, and 1,3-butadiene. Further fractionation will result in fractions consisting principally of butenes, butane and small quantities of butadiene. Other separation of the butadiene by fractionation, for example, is unrewarding but its presence interferes with the general use of these products.

Many different materials have been employed as catalysts for the hydrogenation of various feedstocks in these processes. However, the reaction rate and conversion are frequently not as high as would be desirable. Accordingly, there is a continuing search for catalyst materials which will improve the reaction rate and/or conversion level of these processes.

Accordingly, an object of this invention is to provide a process for the selective removal of contaminants from hydrocarbon streams.

Another object of this invention is to provide a new and improved process for the hydrogenation of unsaturated compounds.

Still another object of this invention is to provide a process for the selective hydrogenation of diolefins in commercial streams containing other unsaturated hydrocarbons.

A further object of this invention is to provide a catalyst effective for the selective hydrogenation of contaminants present in unsaturated hydrocarbon streams.

Other objects, aspects, as well as the several advantages of the invention will be apparent to those skilled in the art upon reading the specification and the appended claims.

## Summary of the Invention

In accordance with the present invention, the selective conversion of diolefins to olefins in the presence of other unsaturated hydrocarbons is accomplished by effecting hydrogenation over a catalyst comprising a Group VIII metal, at least one of lead, tin and germanium, and a support, such as zinc aluminate.

In a process wherein n-butane is converted over a steam active dehydrogenation catalyst to butenes and isobutene in which a small amount of diolefin (butadiene) is made, the improvement comprises passing the stream of butenes mixture along with hydrogen over the same steam active catalyst at lower temperature and shorter residence time whereby the contaminant diolefin (butadiene) is hydrogenated to butenes.

In accordance with one specific embodiment of this invention, a butene stream containing a substantial amount of butene-1, and containing butadiene as an impurity, and which may contain other impurities that can be hydrogenated, is treated with hydrogen and steam under controlled conditions in the presence of a supported Group VIII metal catalyst promoted with at least one of lead, tin and germanium.

The method of this invention is broadly applicable to the selective hydrogenation of olefinic feed stocks. The process is selective because it provides a method of hydrogenating materials such as diolefins and other unsaturated contaminating materials, eg., acetylenic compounds and the like. However, the process is without substantial activity for the hydrogenation of mono-olefins. In some instances, no hydrogenation of mono-olefins is detectable.

## Detailed Description of the Invention

The process of the invention involves passing a hydrocarbon stream containing minor amounts of diolefins and other contaminating materials over a supported Group VIII catalyst promoted with at least one of lead, tin and germanium.

The above catalyst has been found to have a remarkable degree of selectivity in that it is active for the hydrogenation of diolefins to mono-olefins, but relatively inactive for the hydrogenation of mono-olefins to paraffins.

4

The hydrocarbon streams which can be subjected to the process of the invention for hydrotreatment of impurities include any olefinic hydrocarbon-containing stream, especially mono-olefins, which are normally hydrogenatible. A typical stream that is subjected to hydrogenation for removal of unsaturated contaminants according to the invention comprises a butadiene-containing $C_4$ hydrocarbon mixture which contains, for example, butene-1, cis-, and trans-butene-2, n-butane, isobutene and isobutane having a content of butadiene of up to approximately 5 mole percent. Small quantities of other hydrocarbons, for example, $C_3$ and $C_4$ hydrocarbons can also be present. If small quantities of acetylene, such as ethyl or vinylacetylene or compounds with cumulative double bonds, such as propadiene or butadiene-1,2 are present, they will be hydrogenated together with the 1,3-butadiene.

In order to carry out the hydrogenation, hydrogen must be added to the mixture unless it is already present. The quantity of hydrogen required depends on the content of 1,3-butadiene or the compounds to be hydrogenated and must be at least equimolar to them, but can also be a multiple thereof, for example, 5 or 10 times molarwise. Preferably, hydrogen is introduced at a rate which provides a hydrogen to feed molar ratio of from about 0.1 to 1 to about 5 to 1.

The selectivity of the process and catalyst of this invention can be improved by incorporating steam along with the feed stock or by separate introduction into the reaction zone. The steam to hydrocarbon feed molar ratio will range from about 3 to 1 to about 6 to 1. Steam can be introduced with the feed stock or with hydrogen or it can be separately introduced.

The conditions under which the method of this invention is employed can vary widely. Generally, the reaction is conducted by passing the hydrocarbon stream as a vapor with hydrogen and steam into contact with catalyst, the reaction zone being maintained at a temperature from about 400°F to about 700°F, preferably about 500-700°F at a pressure from about 30 to about 60 psig. The hydrocarbon stream is passed in contact with the catalyst at a rate sufficient to provide a liquid hourly space velocity (LHSV) of from about 6 to about 10.

5

The process can be carried out ranging from 1 to 50 atmospheres pressure, preferably from 2 to 30 atmospheres absolute pressure. If the $C_4$ hydrocarbon mixture is not to be further processed in the gaseous state, it is advantageous to operate at hydrogenation pressures of 4 to 8 atmospheres so that the mixture will be in the liquid state at room temperature.

In one particularly preferred embodiment of the invention, hydrogenation of $C_4$ hydrocarbon streams to selectively reduce the amount of diene or other contaminant present is carried out immediately following dehydrogenation of a $C_4$ hydrocarbon stream utilizing the same catalyst. Typical dehydrogenation conditions that can be used are disclosed in U.S. 4,229,609, which is incorporated herein by reference. Generally, the dehydrogenation temperature will range from about 950 to about 1150°F. More specifically, the effluent from dehydrogenation can be passed without separation, but at a lower temperature, over the same steam active catalyst as employed during dehydrogenation. The contact temperature and the residence time during hydrogenation will be substantially less than the same conditions in the dehydrogenation reaction.

The catalyst utilized in all of the embodiments of this invention is broadly a Group VIII metal catalyst on a support. The support can be alumina, silica, magnesia, zirconia, alumina-silicates, Group II aluminate spinels and mixtures of such supports. Group VIII metals are those classified in Group VIII in the Periodic Table of the Elements as set forth in <u>Chemical Rubber Companies</u>, "Handbook of Chemistry and Physics", 45th Edition (1964) page B-2. The noble metals and especially platinum are presently preferred.

The amount of Group VIII metal is not critical. Generally any amount resulting in catalytic activity of the support/metal combination can be utilized. Typically the Group VIII metal is present in the catalyst in an amount in the range of about 0.01 to about 10 parts by weight per 100 parts by weight of support, and frequently the quantity is in the range of about 0.1 to about 5 parts by weight.

Co-promoter metals can be employed in the catalyst in conjunction with the Group VIII metal. The preferred co-promoters are lead, tin and germanium. The co-promoter when employed will be typically used in the range of 0.01 to 10 parts by weight and frequently in a range of 0.1 to 4 parts by weight of co-promoter per 100 parts by weight of support. The co-promoter metals can be employed as chemical compounds such as halides, nitrates, oxylates, acetates, carbonates, propionates, tartrates, bromates, chlorates, oxides, hydroxides, etc. Among the co-promoters, tin is the preferred metal and conveniently and effectively stannous halides can be utilized.

The catalyst used in the processes of this invention are obtained by known methods such as impregnation of the support with the metal compounds. The compounds employed should be such that upon calcination of the catalyst no significant amount of extraneous material remains on the catalyst, particularly no further metals which would interfere with the catalytic process envisaged.

The preferred catalyst useful in the processes of this invention is a catalyst comprising platinum on zinc aluminate, particularly and preferably zinc aluminate spinel. Most preferably the catalyst is co-promoted with tin. Thus, the most preferred catalyst of this invention consists essentially of zinc aluminate spinel, platinum and tin. One typical catalyst can contain about 0.1 to about 5 parts by weight of a zinc aluminate spinel support.

Specific Example

A commercially produced dehydrogenated product comprising $C_4$ hydrocarbons was subjected to selective hydrogenation in the presence of the inventive catalyst to selectively remove diolefins, such as butadiene, from the $C_4$ hydrocarbon feed mixture. The $C_4$ hydrocarbon mixture containing butenes, butane and butadiene in particular, was passed through a hydrogenation reactor at an LHSV of 8 over a catalyst bed comprising 0.6 percent platinum and 1% tin on a zinc aluminate support. The steam to hydrocarbon molar ratio was 5 to 1, and the hydrogenation pressure was 50 psig. The hydrogenation temperature was about 500°F.

The following results were obtained.

TABLE I

| Component | Steam Active Reforming (STAR®) Reactor Feed mol % | Steam Active Reforming (STAR®) Reactor Effluent mol % | Hydrogenation Reactor Effluent mol % |
|---|---|---|---|
| n-butane | 100.0 | 34.74 | 34.75 |
| isobutene | | tr | tr |
| n-butene-1 | | 7.49 | 7.81 |
| t-butene-2 | | 9.04 | 9.44 |
| c-butene-2 | | 7.03 | 7.33 |
| carbon monoxide | | 0.10 | 0.10 |
| carbon dioxide | | 3.01 | 3.01 |
| methane | | 1.82 | 1.82 |
| ethane (and (ethylene) | | 0.38 | 0.38 |
| propane | | 0.65 | 0.65 |
| propylene | | 0.32 | 0.32 |
| butadiene | | 1.02 | <100 ppm |
| pentanes plus (as hexanes) | | 0.04 | 0.04 |
| isobutane | | 0.08 | 0.08 |
| hydrogen | | 34.28 | 34.27 |
| Total | 100.0 | 100.00 | 100.00 |

In this example the butadiene content was reduced from 1.02 weight percent to less than 100 ppm. Thus, high butadiene conversion and simultaneous low butene upon hydrogenation and/or isomerization were demonstrated. In this run, optimization was not made for minimization of concomitant hydrogenation of butenes in the feed to the hydrogenation reactor. Also, although steam was used in the feed, it is believed that steam may not be necessary for the hydrogenation reaction.

PATENT- UND RECHTSANWÄLTE
BARDEHLE · PAGENBERG · DOST · ALTENBURG · FROHWITTER
& PARTNER                    **0211381**

RECHTSANWÄLTE
JOCHEN PAGENBERG DR. JUR. LL. M. HARVARD**
BERNHARD FROHWITTER DIPL.-ING **
JÜRGEN KROHER DR. JUR. LL. M. QUEEN'S UNIV.*

PATENTANWÄLTE · EUROPEAN PATENT ATTORNEYS
HEINZ BARDEHLE DIPL.-ING.
WOLFGANG A. DOST DR., DIPL.-CHEM.
UDO W. ALTENBURG DIPL.-PHYS.
BERNHARD H. GEISSLER DIPL.-PHYS. DR. JUR.
MCL(GWU). AUCH RECHTSANWALT* UND US ATTORNEY AT LAW***

POSTFACH 86 06 20  8000 MÜNCHEN 86
TELEFON (089) 98 03 61
TELEX 522 791 padd
TELEFAX (089) 98 97 63
HYPOBANK MUC 6 860 130 600 (BLZ 700 200 01)
PGA MUC 387 37-808 (BLZ 700 100 80)
BÜRO GALILEIPLATZ 1, 8000 MÜNCHEN 80

DATUM July 29, 1986
31 044-EP

# Claims

1. A process for selectively hydrogenating diolefins present as impurities in hydrocarbon feed streams containing other unsaturated hydrocarbons c h a r a c t e r i z e d b y contacting the feed with hydrogen in the presence of a catalyst comprising a Group VIII metal, at least one of lead, tin and germanium, and a support to obtain a hydrocarbon feed stream wherein the diolefins are selectively hydrogenated without substantial hydrogenation or isomerization of the other unsaturated hydrocarbons present in the feed.

2. The process of claim 1 characterized in that the catalyst comprises platinum, tin and zinc aluminate.

3. The process of claim 1 or 2 characterized in that said contacting temperature ranges from 204 to 371°C, preferably wherein the contacting temperature ranges from 260 to 371°C.

4. The process of any of the preceding claims characterized in that said contacting is carried out in the presence of steam, preferably at a mole ratio of steam to hydrocarbon ranging from 3:1 to 6:1.

5. The process of any of the preceding claims characterized in that said diolefin is butadiene.

6. The process of any of the preceding claims characterized in that said hydrocarbon feed contains butadiene and butene-1.

7. The process of any of the preceding claims characterized in that hydrogen is present in the feed in such an amount to supply the needed hydrogen to selectively hydrogenate butadiene and other contaminants present in the feed material.

8. In a process wherein n-butane is converted at high temperature over a steam active dehydrogenation catalyst to butenes and isobutene in which a small amount of diolefin comprising butadiene is formed, characterized by passing said stream comprising butenes along with hydrogen over the same steam active catalyst at a lower temperature and shorter residence time than during dehydrogenation whereby the contaminant butadiene is hydrogenated to butenes without substantial alteration as by hydrogenation or isomerization of the butenes present in the feed.

9. The process of claim 8 characterized in that the catalyst used for hydrogenation is as defined in any of claims 1 and 2; in particular wherein the hydrogenation conditions are as defined in any of claims 3, 4 and 7.

10. The process of claim 8 or 9 characterized in that the catalyst used for dehydrogenation is the same as that used for hydrogenation.

European Patent Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | US-A-4 152 365 (DREXMAN) <br> * Columns 2,3 * | 1-4 | C 07 C  7/163 <br> C 07 C  11/08 <br> C 07 C  11/09 |
| D,X | US-A-4 229 609 (HUTSON Jr. et al.) <br> * Column 2 * | 9,10 | |
| A | US-A-2 934 574 (C.K. VILAND) | | |

-----

| | |
|---|---|
| | TECHNICAL FIELDS SEARCHED (Int. Cl.4) |
| | C 07 C  7/00 <br> C 07 C  5/00 <br> C 07 C  11/00 |

The present search report has been drawn up for all claims

| Place of search | Date of completion of the search | Examiner |
|---|---|---|
| THE HAGUE | 03-11-1986 | VAN GEYT J.J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82